# EUROPEAN PATENT APPLICATION

(11) **EP 3 025 707 A1**
(43) Date of publication of application: **01.06.2016**
(21) Application number: 15196487.1
(22) Date of filing: 26.11.2015
(51) Int. Cl.: A61K 9/24, A61K 31/155, A61K 31/4439, A61P 3/10

(54) **A MULTILAYER TABLET COMPRISING METFORMIN AND PIOGLITAZONE**

(30) Priority: 27.11.2014 TR 201414137
(71) Applicant: Arven Ilac Sanayi Ve Ticaret A.S., Istanbul 34460 (TR)
(72) Inventor: TÜRKYILMAZ, Ali, 34460 Istanbul (TR); PEHLIVAN AKALIN, Nur, 34460 Istanbul (TR); ZENGINER, Sibel, 34460 Istanbul (TR); AYHAN, Merve, 34460 Istanbul (TR)
(74) Representative: Sevinç, Erkan

(57) **Abstract**

The present invention relates to a multilayer tablet comprising a core comprising metformin hydrochloride, a controlled release layer, an inert layer and a coating layer comprising pioglitazone hydrochloride. The multi-layer tablet is administered once per day to a human in the treatment of Diabetes Mellitus Disease.

## Description

### Field of Invention

The present invention relates to a multilayer tablet comprising a core comprising metformin hydrochloride, a controlled release layer, an inert layer and a coating layer comprising pioglitazone hydrochloride.

### Background of Invention

Diabetes mellitus is a metabolic anomaly of the glucose metabolism occurring, *inter alia*, due to inadequate insulin secretion and decreased sensitivity of insulin target cells and is basically characterized by recognizable hyperglycemia. In the persistent hyperglycemia, severe harmful complications such as retinopathy, nephropathy and neuropathy occur as a result of vascular lesions in various organs and nerves.

Metformin is an oral antidiabetics having an orally-administrated biguanide structure. It is used singly or in combination with sulfonylureas, alpha-glucosidase inhibitors, or insulin. It is freely dissolved in water.

Metformin hydrochloride, with the chemical name 1,1-dimethylbiguanide hydrochloride, has the following chemical structure of Formula I.

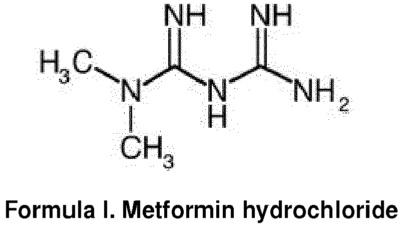

Various patent applications are available in relation to metformin; metformin is disclosed with the patent US 3 174 901 and its prolonged release pharmaceutical formulation is disclosed with US 6 475 521.

Pioglitazone is an orally-administered antidiabetics. It is the member of a group of medications, which are also called as thiazolidinediones and insulin sensitizers. It is not practically soluble in water.

The chemical designation of pioglitazone hydrochloride is (+/-)-5-[[4-[2-(5-ethyl-2-pyridinyl)ethoxy]phenyl]methyl]-2,4-thiazolidinedione hydrochloride, with the following chemical structure of Formula II.

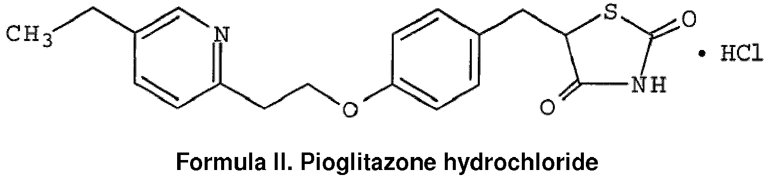

Pioglitazone is disclosed in the patent EP0193256.

Various combination formulations have been disclosed, which comprise metformin and pioglitazone.

The patent EP0514452B2 discloses the use of an active agent selected among pioglitazone hydrochloride, metformin, indole amine antidiabetics, thermogenic β-agonists, CS 045, and thiazolidinedione derivatives, in preparing a medicament for use in treating or preventing hypertension in an insulin-resistant patient.

The application WO03105809 discloses a multilayer tablet composed of thiazolidinedione and biguanide. The tablet comprises immediate-release and controlled-release layers.

Metformin is highly soluble in water. Having high water solubility leads fast dissolution (rapid release) of the drug. In prior art, tablets are known which comprise layers providing immediate release or controlled release. The problem is if, however, the immediate-release layer does not disintegrate rapidly enough, the polymers in the controlled-release layers providing and controlling the release form a diffusion barrier, which completely surrounds the tablet. This barrier prevents the disintegration of the immediate-release layer which does not disintegrate rapidly enough and avoids the active agent from exhibiting its desired activity. Thus, the desired release profile cannot be achieved. Additional time is required for the commencement of activity of currently-available formulations. This is not desirable for the patient. A quick onset of the treatment process directly affects the life quality of patients.

In this novel invention, the active agents in different layers, allows to obtain a desired release profile in the formulation which comprises highly water-soluble metformin and weakly water-soluble pioglitazone. The treatment onset can be realized at a desired level depending particularly on the dissolution rate. Another novelty is carried out with the coating layer including pioglitazone hydrochloride as an active agent. It provides release of the active substance occurs substantially from the surface of the tablet not covered by the one or more barrier layers. Highly water-soluble metformin is in the core and is covered with a controlled release layer. This composition provides to ensure convenient release profiles for pioglitazone hydrochloride and metformin hydrochloride both. And the proportions of the layers in said multilayer tablet are such determined that a formulation is obtained of convenient and desired quality, which provides drug release up to 24 hours.

As a result, based on said drawbacks, a novelty is required in the art of once a day dose formulations containing having metformin hydrochloride and pioglitazone hydrochloride.

### Detailed description of the invention

The multilayer controlled release tablet consisting of one or more barrier layers which limit the release surface of the active ingredients. The layers provide an immediate balance of the blood glucose amount, whereas controlled-release layers provide for a prolonged maintenance of the blood sugar proportion.

The present invention provides a combination formulation of metformin and pioglitazone, eliminating all before said problems and bringing additional advantages to the relevant prior art.

A multilayer tablet comprises; (Figure. I)
a) a core (a) comprising metformin hydrochloride,
b) a controlled release layer (b),
c) an inert layer (c),
d) a coating layer (d) comprising pioglitazone hydrochloride.

According to an embodiment of this invention, the core is present in an amount of 80 to 85 % by weight of the tablet.

According to another embodiment of this invention, the controlled release layer is present in an amount of 1 to 10 % by weight of the tablet.

According to another embodiment of this invention, the inert layer is present in an amount of 1 to 5 % by weight of the tablet.

According to another embodiment of this invention, the coating layer is present in an amount of 1 to 10 % by weight of the tablet.

In a preferred embodiment according to the present invention, the core further comprises polyvinylpyrrolidone, hydroxypropyl methylcellulose, sodium lauryl sulfate, polyethylene glycol and magnesium stearate.

In an embodiment of the present invention, the controlled release layer comprises poly(meth)acrylate (Eudragit® which is the aqueous dispersion of a neutral copolymer based on ethyl acrylate and methyl methacrylate), Opadry® white (is mixture of polyvinyl alcohol, titanium dioxide, polyethylene glycol, talc), simethicone emulsion and hydroxypropyl methylcellulose.

According to an embodiment of this invention, the inert layer comprises polyvinyl alcohol, titanium dioxide, polyethylene glycol and talc.

According to another embodiment of this invention, the coating layer further comprises lactose monohydrate, hydroxypropyl methylcellulose, polyethylene glycol and poly(meth)acrylate (Eudragit®which is copolymer of ethyl acrylate, methyl methacrylate and a low content of methacrylic acid ester with quaternary ammonium groups).

The selection and proportion of the excipients, which are to provide the desired release profile are quite critical. In this present invention to adjust the dissolution profile, Eudragit® and simethicone emulsion has been added as controlled release agents. The amount of Eudragit® and simethicone emulsion in different layers, allows to obtain a desired release profile in the formulation which comprises highly water-soluble metformin and weakly water-soluble pioglitazone. The treatment onset can be realized at a desired level depending particularly on the dissolution rate.

In a preferred embodiment according to the present invention, the content of metformin hydrochloride is 1000 mg.

In another preferred embodiment according to the present invention, the content of pioglitazone hydrochloride is 30 mg.

According to another embodiment of this invention, the release of metformin hydrochloride at the end of 2, 8 and 20 hours lies in the range of maximum 25%, 35-70% and minimum 80% respectively. In other words, metformin hydrochloride releases in the range of max. 25% at the end of 2 hours and releases in the range of 35-70% at the end of 8 hours and releases in the range of min. 80% at the end of 20 hours.

According to another embodiment of this invention, the release of pioglitazone hydrochloride at the end of 30 minutes, 2 and 16 hours lies in the range of 10-32%, 35-65% and minimum 80% respectively. In other words, pioglitazone hydrochloride releases in the range of 10-32% at the end of 30minutes and releases in the range of 35-65% at the end of 2 hours and releases in the range of minimum 80% at the end of 16 hours.

In a preferred embodiment according to the present invention, the multi-layer tablet comprises;
a. a core (a) comprising metformin hydrochloride as drug substance and;
   i. 5 to 10% by weight of polyvinylpyrrolidone,
   ii. 7 to 12 % by weight of hydroxypropyl methylcellulose,
   iii. 0.5 to 5 % by weight of sodium lauryl sulfate,
   iv. 0.5 to 5 % by weight of polyethylene glycol,
   v. 0.2 to 4 % by weight of magnesium stearate.
b. a controlled release layer (b) comprising;
   i. 58 to 65 % by weight of poly(meth)acrylate (Eudragit® which is the aqueous dispersion of a neutral copolymer based on ethyl acrylate and methyl methacrylate),
   ii. 28 to 35 % by weight of Opadry® white (mixture of polyvinyl alcohol, titanium dioxide, polyethylene glycol, talc);
   iii. 0.1 to 5 % by weight of simethicone emulsion,
   iv. 5 to 10 % by weight of hydroxypropyl methylcellulose.
c. an inert layer (c) provided between the controlled release layer (b) and the coating layer (d) comprising the mixture of polyvinyl alcohol, titanium dioxide, polyethylene glycol, talc.
d. a coating layer (d) comprising pioglitazone hydrochloride as drug substance and;
   i. 8 to 15 % by weight of lactose monohydrate,
   ii. 28 to 38 % by weight of hydroxypropyl methylcellulose,
   iii. 10 to 15 % by weight of polyethylene glycol,
   iv. 8 to 18 % by weight of poly(meth)acrylate (Eudragit® which is a copolymer of ethyl acrylate, methyl methacrylate and a low content of methacrylic acid ester with quaternary ammonium groups).

According to an embodiment of this invention, the multi-layer tablet is for use in the treatment of Diabetes Mellitus Disease.

According to another embodiment of this invention, the multi-layer tablet is administered once per day to a human in need of such treatment.

The present invention is to reduce the treatment onset time with said formulation providing a 24-hour effect.

### Example:

| **Core** | **amount (%)** |
|---|---|
| Metformin HCl | 80 - 85 |
| Polyvinylpyrrolidone (PVP K-90) | 5 - 10 |
| Hydroxypropyl methylcellulose (HPMC E5 LV) | 7 - 12 |
| Sodium lauryl sulfate | 0.5 - 5 |
| Polyethylene glycol (PEG 6000) | 0.5 - 5 |
| Magnesium stearate | 0.2 - 4 |

| **Controlled release layer** | **amount (%)** |
|---|---|
| Eudragit® NE 30D | 58 - 65 |
| Opadry white (85F18422) * | 28 - 35 |
| Simethicone emulsion | 0.1 - 5 |
| Hydroxypropyl methylcellulose (HPMC E15 LV) | 5 - 10 |
| Distilled water | q.s |

| **Inert layer** | **amount (%)** |
|---|---|
| Opadry white (85F18422) (%18) * | 100 |
| Distilled water | q.s |

| **Coating layer** | **amount (%)** |
|---|---|
| Pioglitazone HCl (6.8% solution) (10% excess) | 30 - 40 |
| Lactose monohydrate | 8 - 15 |
| Hydroxypropyl methylcellulose (HPMC E5 LV) | 18 - 23 |
| Hydroxypropyl methylcellulose (HPMC E15 LV) | 10 - 15 |
| Polyethylene glycol (PEG 4000) | 10 - 15 |
| Eudragit® RS 30% | 4 - 9 |
| Eudragit® RL 30% | 4 - 9 |
| Distilled water | q.s |
| | |

| ***Opadry white (85F18422)** | **amount (%)** |
|---|---|
| Polyvinyl alcohol (PVA) | 38 - 44 |
| Titanium dioxide | 22 - 27 |
| Polyethylene glycols (PEG 3350 powder) | 18 - 24 |
| Talc | 12 - 18 |

**Production process of core:** Weighed amount of metformin hydrochloride is charged to a granulator and mixed. Hydroxypropyl methylcellulose (HPMC E5 LV), polyvinylpyrrolidone (PVP K-90) and sodium lauryl sulfate are added and mixed. Distilled water is sprayed over the mixture present in the granulator. The prepared wet granules are sieved and are added to the fluidized bed dryer and dried. The dried granules are passed through a sieve and ground. Polyethylene glycol (PEG 6000) is added to the sieved dry granules and mixed for around 10 minutes. Magnesium stearate is added into this mixture and is mixed for around 3 more minutes. The final mixture is compressed as tablets.

**Production process of controlled release layer:** Hydroxypropyl methylcellulose (HPMC E15 LV) is mixed with distilled water for 15 minutes. opadry white (85F18422) is added and mixed for 5 minutes. Eudragit® NE 30D and simethicone emulsion are added to the solution and mixed for 45 minutes. The final mixture is sprayed over the core as a controlled release layer.

**Production process of inert layer:** Opadry white (85F18422) is mixed with distilled water to make a 18% (w/w) solution. The final solution is sprayed over the tablet as an inert layer.

**Production process of coating layer:** Weighed amounts of pioglitazone hydrochloride, lactose monohydrate, hydroxypropyl methylcellulose (HPMC E5 LV), hydroxypropyl methylcellulose (HPMC E15 LV) and polyethylene glycol (PEG 4000) are mixed together. Eudragit® RS 30% and Eudragit® RL 30% are added to the distilled water and mixed. The first solid mixture with pioglitazone hydrochloride is added to the aqueous solution and mixed. The final solution is sprayed over the tablet as a coating layer.

### Dissolution profile of the multilayer tablet:

The final dosage form is taken to the vessels for testing the release profile in the gastric environment.

### Method:

| | |
|---|---|
| **Method** | : Paddle |
| **Rotation speed** | : 50 rpm/min |
| **Time** | : 1200 minutes |
| **Temperature** | : 37 °C ± 0.5 °C |
| **Dissolution medium** | : 900 mL, 0.1 M Citric acid buffer, pH 2.5 |

| **Time (min.)** | **Metformin HCl / Pioglitazone HCl 1000/30 XR Tablet** | |
|---|---|---|
| | **Metformin HCl** | **Pioglitazone HCl** |
| **5** | - | 11 |
| **10** | - | 16 |
| **15** | - | 19 |
| **20** | - | 20 |
| **30** | - | 24 |
| **45** | - | 31 |
| **60** | 2 | 37 |
| **120** | 6 | 46 |
| **180** | 11 | 54 |
| **240** | 17 | 63 |
| **300** | 26 | 64 |
| **360** | 37 | 68 |
| **480** | 53 | 79 |
| **600** | 65 | 82 |
| **720** | 75 | 86 |
| **960** | 89 | 90 |
| **1200** | 97 | 92 |
| **1440** | 102 | 94 |

**Results:** The proportions of the layers in said multiayer tablet are such determined that a formulation is obtained of convenient and desired quality, which provides drug release up to 24 hours.

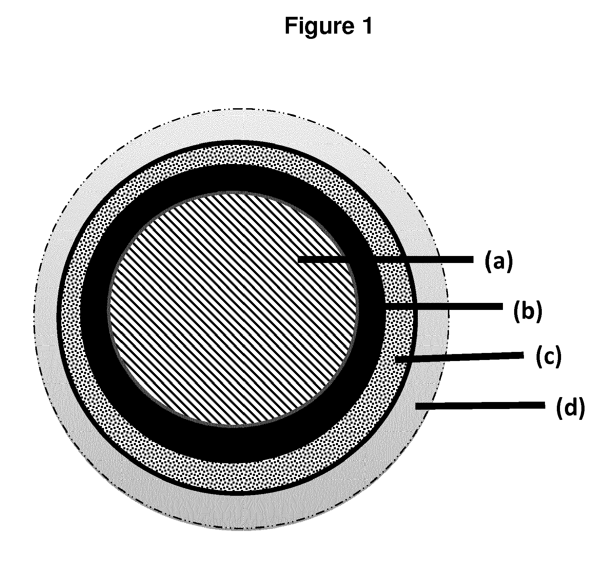

## Claims

1. A multilayer tablet comprising;
a) a core comprising metformin hydrochloride,
b) a controlled release layer,
c) an inert layer
d) a coating layer comprising pioglitazone hydrochloride.

2. The multilayer tablet according to claim 1, wherein the core is present in an amount of 80 to 85 % by weight of the tablet.

3. The multilayer tablet according to claim 1, wherein the controlled release layer is present in an amount of 1 to 10 % by weight of the tablet.

4. The multilayer tablet according to claim 1, wherein the inert layer is present in an amount of 1 to 5 % by weight of the tablet.

5. The multilayer tablet according to claim 1, wherein the coating layer is present in an amount of 1 to 10 % by weight of the tablet.

6. The multilayer tablet according to claim 1, wherein the core further comprising polyvinylpyrrolidone, hydroxypropyl methylcellulose, sodium lauryl sulfate, polyethylene glycol and magnesium stearate.

7. The multilayer tablet according to claim 1, wherein the controlled release layer comprising poly(meth)acrylates, polyvinyl alcohol, titanium dioxide, polyethylene glycol, talc, simethicone and hydroxypropyl methylcellulose.

8. The multilayer tablet according to claim 1, wherein the inert layer comprising polyvinyl alcohol, titanium dioxide, polyethylene glycol and talc.

9. The multilayer tablet according to claim 1, wherein the coating layer further comprising lactose monohydrate, hydroxypropyl methylcellulose, polyethylene glycol and poly(meth)acrylates.

10. The multilayer tablet according to claim 2, wherein the content of metformin hydrochloride is 1000 mg.

11. The multilayer tablet according to claim 5, wherein the content of pioglitazone hydrochloride is 30 mg.

12. The multilayer tablet according to claim 1, wherein the release of metformin hydrochloride at the end of 2, 8 and 20 hours lies in the range of maximum 25%, 35-70% and minimum 80% respectively.

13. The multilayer tablet according to claim 1, wherein the release of pioglitazone hydrochloride at the end of 30minutes, 2 and 16 hours lies in the range of 10-32%, 35-65% and minimum 80% respectively.

14. The multi-layer tablet according any to the preceeding claims, wherein the tablet comprising;
a. a core (a) comprising metformin hydrochloride as drug substance and;
i. 5 to 10% by weight of polyvinylpyrrolidone,
ii. 7 to 12 % by weight of hydroxypropyl methylcellulose,
iii. 0.5 to 5 % by weight of sodium lauryl sulfate,
iv. 0.5 to 5 % by weight of polyethylene glycol,
v. 0.2 to 4 % by weight of magnesium stearate.
b. a controlled release layer (b) comprising;
i. 58 to 65 % by weight of poly(meth)acrylate,
ii. 28 to 35 % by weight of mixture of polyvinyl alcohol, titanium dioxide, polyethylene glycol, talc,
iii. 0.1 to 5 % by weight of simethicone emulsion,
iv. 5 to 10 % by weight of hydroxypropyl methylcellulose.
c. an inert layer (c) provided between the controlled release layer (b) and the coating layer (d) comprising the mixture of polyvinyl alcohol, titanium dioxide, polyethylene glycol and talc.
d. a coating layer (d) comprising pioglitazone hydrochloride as drug substance and;
i. 8 to 15 % by weight of lactose monohydrate,
ii. 28 to 38 % by weight of hydroxypropyl methylcellulose,
iii. 10 to 15 % by weight of polyethylene glycol,
iv. 8 to 18 % by weight of poly(meth)acrylate.

15. The multi-layer tablet according to any of the preceeding claims, for use in the treatment of Diabetes Mellitus Disease.

16. The multi-layer tablet according to any of the preceeding claims, wherein said tablet is administered once per day to a human in need of such treatment.
